# EUROPÄISCHE PATENTANMELDUNG

(11) **EP 2 803 660 A1**
(43) Veröffentlichungstag der Anmeldung: **19.11.2014**
(21) Anmeldenummer: 13167511.8
(22) Anmeldetag: 13.05.2013
(51) Int. Cl.: C07C 271/28, C07C 275/40, C08G 18/02

(54) **Neue Carbodiimide mit endständigen Harnstoff- und/oder Urethangruppen, Verfahren zu deren Herstellung und deren Verwendung**

(71) Anmelder: Rhein Chemie Rheinau GmbH, 68219 Mannheim (DE)
(72) Erfinder: Laufer, Wilhelm, 67158 Ellerstadt (DE); Bechem, Benjamin, 68157 Mannheim (DE); Eckert, Armin, 68794 Oberhausen-Rheinhausen (DE)
(74) Vertreter: Siegers, Britta

(57) **Zusammenfassung**

Die Erfindung betrifft neue Carbodiimide mit endständigen Harnstoff- und/oder Urethangruppen, Verfahren zu deren Herstellung und deren Verwendung als Stabilisator in esterbasierten Polyolen, in thermoplastischen Polyurethanen, in Polyurethan-Elastomeren, in PU-Klebstoffen, in PU-Gießharzen, für PU-Beschichtungen oder in PU-Schäumen.

## Beschreibung

Die Erfindung betrifft neue Carbodiimide mit endständigen Harnstoff- und/oder Urethangruppen, Verfahren zu deren Herstellung und deren Verwendung als Stabilisator in esterbasierten Polyolen, in thermoplastischen Polyurethanen, in Polyurethan-Elastomeren, in PU-Klebstoffen, in PU-Gießharzen, für PU-Beschichtungen oder in PU-Schäumen.

Carbodiimide haben sich in vielen Anwendungen bewährt, z.B. als Hydrolyseschutzmittel für Thermoplasten, Polyole, Polyurethane, Triglyceride und Schmierstofföle etc.

Bevorzugt werden hierfür sterisch gehinderte aromatische Monocarbodiimide eingesetzt. Bekannt ist in diesem Zusammenhang vor allem 2,6-Diisopropylphenyl-Carbodiimid. Diese Monocarbodiimide haben jedoch den Nachteil, selbst bei niedrigen Temperaturen flüchtig zu sein. Sie sind thermisch nicht stabil und können giftige, flüchtige Substanzen abspalten. Weitere Carbodiimide, wie in EP 0 628 541 A1 beschrieben, basieren auf speziellen Rohstoffen, die sehr teuer in der Anschaffung sind. Zudem haben diese bei Raumtemperatur hohe Viskositäten, die den Umgang mit diesen Carbodiimiden erschweren. Weiterhin ist in bestimmten PU-Anwendungen deren Reaktivität und/oder deren stabilisierende Wirkung bei den standardmäßig eingesetzten Konzentrationen nicht ausreichend. Polymere Carbodiimide, die auf preiswerten Rohstoffen basieren, wie in DE-A 2248751 und US-A 2941983 beschrieben, sind nicht ausreichend sterisch gehindert und zeigen in den meisten esterbasierten Polymeren keine gute Hydrolyseschutzwirkung. Stark sterisch gehinderte Carbodiimide, wie z.B. solche auf Basis Triisopropylphenylisocyanat, sind sehr wirksam, haben jedoch sehr hohe Schmelzpunkte, sind nicht löslich und können nicht oder nur mit einem beträchtlichen apparativen und zeitlichen Aufwand in die Ausgangsstoffe der Polyurethane eingebracht werden.

Es besteht daher ein Bedarf an neuen Carbodiimide, welche die Nachteile des Standes der Technik nicht aufweisen, über eine hohe thermische Stabilität verfügen und zum Hydrolyseschutz esterbasierter Polymere eingesetzt werden können.

Überraschenderweise konnte diese Aufgabe durch den Einsatz von bestimmten aromatischen Carbodiimide gelöst werden.

Gegenstand der vorliegenden Erfindung sind daher Carbodiimide mit endständigen Harnstoff- und oder Urethangruppen der Formel (I) 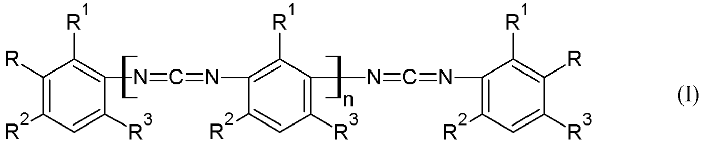 in der
- R gleich oder verschieden sein kann und ausgewählt ist aus der Gruppe der -NHCONHR^{I}-,
   - NHCONR^{I}R^{II}- und -NHCOOR^{III}-Reste, wobei R^{I} und R^{II} gleich oder verschieden sind und einen C₁ - C₂₂ - Alkyl-, C₆ - C₁₂ -Cycloalkyl-, C₆ - C₁₈ -Aryl- oder C₆ - C₁₈ -Aralkylrest bedeuten und R^{III} gleich C₁ - C₂₂ - Alkyl, C₆ - C₁₂ -Cycloalkyl, C₆ - C₁₈ -Aryl oder C₆ - C₁₈
   - Aralkyl, sowie einen ungesättigten Alkylrest (z.B. einen Oleylrest) mit 2 - 22, bevorzugt 12
   - 20, besonders bevorzugt 16 - 18 Kohlenstoffatomen, oder ein Alkoxypolyoxyalkylenrest bedeutet,
- R¹, R² und R³ jeweils unabhängig für Methyl- oder Ethyl steht, wobei jeder Benzolring nur eine Methyl-Gruppe aufweist und
- n = 0 bis 10, bevorzugt n = 1 bis 4, besonders bevorzugt n = 1 bis 3 ist.

Bevorzugt sind Carbodiimide der Formel (I) mit R = -NHCOOR^{III}, wobei R^{III} ein Alkoxypolyoxyalkylenrest ist, R¹, R² und R³ jeweils unabhängig für Methyl- oder Ethyl steht, wobei jeder Benzolring nur eine Methyl-Gruppe aufweist und n = 0 bis 10, bevorzugt n = 1 bis 4, besonders bevorzugt n = 1 bis 3.

Bevorzugte Alkoxypolyoxyalkylenreste sind Polyethylenglykolmonomethylether mit Molmassen von 200 - 600 g/mol, besonders bevorzugt von 350 - 550 g/mol.

In einer weiteren Ausführung der Erfindung sind solche Carbodiimide der Formel (I) bevorzugt, in denen R gleich -NHCOOR^{III} ist, wobei R^{III} ein gesättigter und/oder ungesättigter Alkylrest mit 2 - 22, bevorzugt 12 - 20, besonders bevorzugt 16 - 18 Kohlenstoffatomen ist, und R¹, R² und R³ jeweils unabhängig für Methyl- oder Ethyl steht, wobei jeder Benzolring nur eine Methyl-Gruppe aufweist und n = 0 bis 10, bevorzugt n = 1 bis 4, besonders bevorzugt n =1 bis 3 ist.

Der Carbodiimid-Gehalt (NCN-Gehalt, gemessen durch Titration mit Oxalsäure) der erfindungsgemäßen Carbodiimide liegt vorzugsweise bei 2 - 10 Gew.%, bevorzugt 4 - 8 Gew.%, besonders bevorzugt bei 5 - 7 Gew.%.

Die erfindungsgemäßen Carbodiimide weisen darüber hinaus vorzugsweise mittlere Molmassen (Mw) von 1000 - 5000 g/mol, bevorzugt 1500 - 4000 g/mol, besonders bevorzugt 2000 - 3000 g/mol auf.

Weiterhin sind Carbodiimide bevorzugt, die eine Polydispersität D = Mw/Mn von 1,2 - 2, besonders bevorzugt von 1,4 - 1,8 aufweisen.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Gegenstand der vorliegenden Erfindung ist zudem die Herstellung der erfindungsgemäßen Carbodiimide durch die Carbodiimidisierung von aromatischen Diisocyanaten der Formel (II) 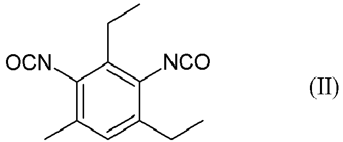 und/oder der Formel (III) 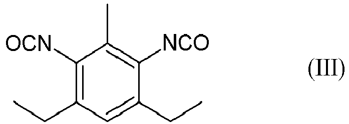 unter Abspaltung von Kohlendioxid bei Temperaturen von 80 °C bis 200 °C in Gegenwart von Katalysatoren und gegebenenfalls Lösungsmittel und der anschließenden Endfunktionalisierung der freien NCO-Gruppen mit primären oder sekundären Aminen oder Alkoholen und/oder Alkoxypolyoxyalkylenalkoholen.

Bevorzugt werden Mischungen der Diisocyanate der Formeln (II) und (III) eingesetzt, vorzugsweise im Verhältnis 60 : 40 bis 95 : 5, besonders bevorzugt 70 : 30 bis 90 : 10.

Die für die Herstellung der Diisocyanate notwendigen aromatischen Diamine können - wie dem Fachmann bekannt- über eine Friedel-Crafts Alkylierung der entsprechenden Toluoldiamine mit dem entsprechenden Alken, oder Halogenalkan hergestellt werden. Bei den aromatischen Diaminen handelt es sich um handelsübliche Verbindungen, die z.B. bei der Firma Lonza AG unter dem Handelsnamen Lonzacure® erhältlich sind.

Anschließend werden diese Diamine mit Phosgen zum entsprechenden Diisocyanat umgesetzt.

Zur Herstellung der erfindungsgemäßen Carbodiimide können die Diisocyanate der Formel (II) und/oder (III) bei erhöhter Temperaturen, vorzugsweise Temperaturen von 80 - 200 °C, besonders bevorzugt von 100 bis 180°C, ganz besonders bevorzugt von 120 - 140 °C, zweckmäßigerweise in Gegenwart von Katalysatoren unter Kohlendioxidabspaltung kondensiert werden. Hierfür geeignete Verfahren werden beispielsweise beschrieben in DE-A 1130594 und DE-A 11564021.

Als Katalysatoren für die Herstellung der Verbindungen der Formel (I) sind in einer Ausführungsform der Erfindung Phosphorverbindungen bevorzugt. Als Phosphorverbindungen werden vorzugsweise Phospholenoxide, Phospholidine oder Phospholinoxide sowie die entsprechenden Phospholensulfide verwendet. Ferner können als Katalysatoren tertiäre Amine, basisch reagierende Metallverbindungen, Alkali-, Erdalkalioxide oder -Hydroxide, -Alkoholate oder - Phenolate, Carbonsäuremetallsalze und nicht basische Organometallverbindungen verwendet werden.

Die Carbodiimidisierung kann sowohl in Substanz als auch in einem Lösemittel durchgeführt werden. Als Lösemittel werden vorzugsweise Alkylbenzole, Paraffinöle, Polyethylenglykoldimethylether, Ketone oder Lactone eingesetzt.

Wenn die Reaktionsmischung den gewünschten Gehalt an NCO-Gruppen, entsprechend einem mittleren Kondensationsgrad n von 0 bis 10, bevorzugt 1 - 4, besonders bevorzugt 1 - 3, besitzt wird die Polycarbodiimidisierung üblicherweise gestoppt. Hierzu wird die Temperatur der Reaktionsmischung auf 50 - 120 °C, bevorzugt 60 - 100 °C, besonders bevorzugt auf 80 - 90 °C reduziert und die Katalysatoren werden unter verminderten Druck abdestilliert. In einer bevorzugten Herstellungsvariante der erfindungsgemäßen Carbodiimide wird anschließend das überschüssige Diisocyanat bei Temperaturen von 150 - 200° C, bevorzugt 160 - 180°C abdestilliert. Anschließend werden die freien endständigen Isocyanatgruppen der Carbodiimide mit aliphatischen und/oder aromatischen Aminen, Alkoholen und/oder Alkoxypolyoxyalkylenalkoholen vorzugsweise in einem geringem Überschuss an -NH-, -NH₂- und/oder -OH-Gruppen, gegebenenfalls in Anwesenheit eines dem Fachmann bekannten PU-Katalysators, vorzugsweise tert. Aminen oder OrganozinnVerbindungen, besonders bevorzugt DBTL (Dibutylzinn dilaurat oder DOTL (Dioctylzinndilaurat), abreagiert. Das Verhältnis von Aminen, Alkoholen und/oder Alkoxypolyoxyalkylenalkoholen zu Carbodiimiden liegt vorzugsweise bei 1,005 - 1,05 : 1, besonders bevorzugt bei 1,01 - 1,03 : 1, bezogen auf die N=C=O-Gruppen.

Vorzugsweise werden die erfindungsgemäßen Carbodiimide nach ihrer Herstellung gereinigt. Die Reinigung der Rohprodukte kann destillativ und/oder mittels Extraktion mit Lösemitteln erfolgen. Als geeignete Lösemittel für die Aufreinigung können vorzugsweise Polyethylenglykoldimethylether, Alkylbenzole, Paraffinöle, Alkohle, Ketone oder Ester eingesetzt werden. Dabei handelt es sich um handelsübliche Lösemittel.

Gegenstand der vorliegenden Erfindung ist weiterhin eine Zusammensetzung enthaltend
- mindestens esterbasiertes Polymer, vorzugsweise ausgewählt aus der Gruppe der Polyesterpolyole, der esterbasierten thermoplastischen Polyurethane, der esterbasierten Polyurethan-Elastomere oder -Schäume,
   und
- mindestens ein erfindungsgemäßes Carbodiimid der Formel (I).

Dabei beträgt die Konzentration des erfindungsgemäßen Carbodiimides der Formel (I) in der erfindungsgemäßen Zusammensetzung vorzugsweise 0,1 - 5 Gew.%, bevorzugt 0,5 - 3 Gew.%, besonders bevorzugt 1 - 2 Gew.%.

Bei den Polyesterpolyolen als esterbasierte Polymere handelt es sich vorzugsweise um langkettige Verbindungen, die vorzugsweise ein Molekulargewicht (in g/mol) von bis zu 2000, bevorzugt zwischen 500 - 2000 und besonders bevorzugt zwischen 500 - 1000, aufweisen.

Der Begriff Polyesterpolyole im Sinne der Erfindung umfasst dabei sowohl langkettige Diole als auch Triole, wie auch Verbindungen mit mehr als drei Hydroxylgruppen je Molekül.

Vorteilhaft ist es, wenn das Polyesterpolyol eine OH-Zahl von bis zu 200, vorzugsweise zwischen 20 und 150 und besonders bevorzugt zwischen 50 und 115, aufweist. Insbesondere eignen sich Polyesterpolyole, die Reaktionsprodukte von verschiedenen Polyolen mit aromatischen oder aliphatischen Dicarbonsäuren und/oder Polymere von Lactonen sind.

Bei den im Sinne der Erfindung eingesetzten Polyesterpolyolen handelt es sich um handelsübliche Verbindungen, die bei der Firma Bayer MaterialScience AG unter dem Handelsnamen Baycoll® oder Desmophen® erhältlich sind.

Gegenstand der vorliegenden Erfindung ist zudem die Verwendung der erfindungsgemäßen Carbodiimide in esterbasierten Polyolen, in Triglyceriden, vorzugsweise Trimethylolpropantrioleat (TMP-Oleat), in Ölformulierungen für die Schmierstoffmdustrie, in thermoplastischen Polyurethanen (TPU), in Polyurethan-Elastomeren, in PU-Klebstoffen, in PU-Gießharzen, in PU-Schäumen oder in PU-Beschichtungen für Holz, Leder, Kunstleder und Textilien als Schutz gegen hydrolytischen Abbau.

Die nachfolgenden Beispiele dienen der Erläuterung der Erfindung, ohne dabei limitierend zu wirken.

### Ausführungsbeispiele

Getestet wurden:
1) CDI (A): ein flüssiges Carbodiimid mit einem NCN-Gehalt von ca. 7,5 Gew.%, auf Basis von 1,3-Bis-(1-methyl-1-isocyanatoethyl)-benzol, endfunktionalisiert mit Polyethylenglykol-monomethylether PEG 550 MME, hergestellt analog zu Beispiel 2 in EP-A 0 628 541.
2) CDI (B): ein flüssiges Carbodiimid der Formel (I) mit einem NCN-Gehalt von ca. 6 Gew.%, erhalten durch Umsetzung einer Mischung auf Basis ca. 80 Gew.% von 2,4-Diethyltoluoyldiisocyanat, d.h. des Diisocyanats der Formel (II), und 20 Gew.% von 2,6-Diethyltoluoyldiisocyanat, d.h. des Diisocyanats der Formel (III), endfunktionalisiert mit Polyethylenglykol-monomethylether (PEG 550 MME der Firma BASF SE).

### Herstellung des erfindungsgemäßen Carbodiimides (CDI B)

In einem ausgeheizten und mit Stickstoff befüllten 250 ml Vierhalskolben wurden unter Stickstoffstrom 92 g einer Mischung, bestehend aus ca. 20 Gew.% 2,6 Diethyltoluoyldiisocyanat und ca. 80 Gew.% 2,4-Diethyltoluoyldiisocyanat vorgelegt. Nach Zugabe von 50 mg 1-Methylphospholenoxid wurde auf 130 °C erwärmt. Im Anschluss wurde bei 130 °C solange reagiert bis ein NCO-Gehalt von ca. 12 Gew.% erreicht worden war. Nachdem der Katalysator bei einer Temperatur von ca. 120 °C unter Vakuum vollständig destillativ entfernt wurde, wurde das überschüssige Diisocyanat bei einer Temperatur von ca. 180 °C unter Vakuum abdestilliert. Schließlich wurde die Reaktionsmischung auf ca. 90 - 100°C gekühlt und die endständigen NCO-Gruppen mit PEG 500 MME abreagiert. Das erhaltene Produkt war eine helle, gelbe Flüssigkeit mit einem NCN-Gehalt von ca. 6 Gew.% (d. h. n = 1,7) und einer Polydispersität (Mw/Mn) von ca. 1,5.

### Thermische Stabilität

Zur Untersuchung der thermischen Stabilität wurden bei 80 °C 1 Gew.% der oben genannten Carbodiimide in Polyesterpolyol mit einer Anfangssäurezahl von ca. 0,9 mg KOH/g eingerührt und 3 h bei dieser Temperatur weiter gerührt. Die Gasphase wurde mittels GC-MS auf Spaltsubstanzen (Isocyanate) untersucht.

Die Ergebnisse werden in Tabelle 1 dargestellt:

| **Carbodiimid** | **Nachweis von Spaltsubstanzen (Isocyanate)** |
|---|---|
| CDI (A) (V) | Negativ |
| CDI (B) (erf) | Negativ |

| | |
|---|---|
| V = Vergleichsbeispiel, erf = erfindungsgemäß | |

Aus der Tabelle 1 ist ersichtlich, dass das erfindungsgemäße Carbodiimid eine hervorragende thermische Stabilität aufweist, die mit CDI (B) vergleichbar ist.

### Säurezahlabbau in Polyesterpolyol

Wie bekannt, kann die Wirkung eines Hydrolyseschutzmittels auf Basis sterisch gehinderter Carbodiimide in flüssigen Polyesterpolyolen mittels Säurezahlabbau geprüft werden.

Der Säurezahlabbau in der erfindungsgemäßen Zusammensetzung wurde beim Einsatz des erfindungsgemäßen CDI (B) im Vergleich zu dem aus dem Stand der Technik bekannten CDI (A) einem Polyesterpolyol auf Basis von Adipinsäure (Desmophen® 2001 KS der Bayer MaterialScience AG) geprüft.

Dazu wurden bei 80°C 1 Gew.% der oben genannten Carbodiimide in Polyesterpolyol mit einer anfänglichen Säurezahl von ca. 0,9 mg KOH/g eingerührt und in regelmäßigen Abständen die Säurezahl gemessen.

Die Ergebnisse werden in Tabelle 2 dargestellt:

| **Carbodiimid in Desmophen ® 2001 KS** | **Säurezahl [mg KOH/g] nach 0 min** | **Säurezahl [mg KOH/g] nach 30 min** | **Säurezahl [mg KOH/g] nach 60 min** | **Säurezahl [mg KOH/g] nach 120 min** | **Säurezahl [mg KOH/g] nach 240 min** | **Säurezahl [mg KOH/g] nach 480 min** |
|---|---|---|---|---|---|---|
| **CDI (B) (erf)** | 0,86 | 0,63 | 0,52 | 0,38 | 0,21 | 0,12 |
| **CDI (A) (V)** | 0,87 | 0,69 | 0,55 | 0,42 | 0,35 | 0,28 |

| | | | | | | |
|---|---|---|---|---|---|---|
| V = Vergleichsbeispiel, erf = erfindungsgemäß | | | | | | |

Die Ergebnisse zeigen, dass die Restsäure des Polyesterpolyols mit dem erfindungsgemäßen Carbodiimid trotz des geringeren NCN-Gehaltes überraschenderweise deutlich schneller abgebaut wird als bei der Zusammensetzung, die das nach dem Stand der Technik bekannt CDI (A) enthält.

## Patentansprüche

1. Carbodiimide mit endständigen Harnstoff- und/oder Urethangruppen der Formel 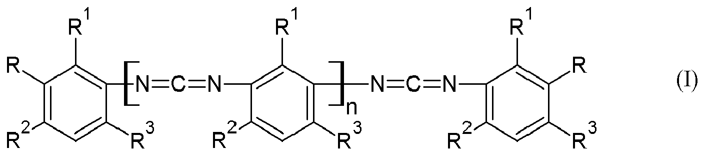
- in der R gleich oder verschieden sein kann und ausgewählt ist aus der Gruppe der
- NHCONHR^{I}-, -NHCONR^{I}R^{II}- und -NHCOOR^{III}-Reste, wobei R^{I} und R^{II} gleich oder verschieden sind und einen C₁ - C₂₂ - Alkyl-, C₆ - C₁₂ -Cycloalkyl-, C₆ - C₁₈-Aryl- oder C₆ - C₁₈ -Aralkylrest bedeuten und R^{III} C₁ - C₂₂ - Alkyl, C₆ - C₁₂ - Cycloalkyl, C₆ - C₁₈ -Aryl oder C₆ - C₁₈ -Aralkyl, oder einem ungesättigten Alkylrest mit 2-22, bevorzugt 12-20, besonders bevorzugt 16-18 Kohlenstoffatomen, oder einem Alkoxypolyoxyalkylenrest entspricht,
- R¹, R² und R³ jeweils unabhängig für Methyl- oder Ethyl steht, wobei jeder Benzolring nur eine Methyl-Gruppe aufweist und
- n = 0 bis 10 bedeutet.

2. Carbodiimide nach Anspruch 1, **dadurch gekennzeichnet, dass** R ein -NHCOOR^{III}-Rest mit R^{III} ein Alkoxypolyoxyalkylen ist, und R¹, R² und R³ jeweils unabhängig für Methyl- oder Ethyl steht, wobei jeder Benzolring nur eine Methyl-Gruppe aufweist und n = 0 bis 10 bedeutet.

3. Carbodiimide nach Anspruch 1 oder 2, **dadurch gekennzeichnet, dass** R ein -NHCOOR^{III}-Rest, wobei R^{III} ein ungesättigter Alkylrest mit 18 Kohlenstoffatomen (Oleylrest) ist, und R¹, R² und R³ jeweils unabhängig für Methyl- oder Ethylsteht, wobei jeder Benzolring nur eine Methyl-Gruppe aufweist und n = 0 bis 10 bedeutet.

4. Carbodiimide nach einem der Ansprüche 1 bis 2, **dadurch gekennzeichnet, dass** R^{III} ein Polyethylenglykolmonomethylether mit Molmassen von 200 - 600 g/mol, bevorzugt von 350 - 550 g/mol ist.

5. Carbodiimide nach einem der Ansprüche 1 bis 4, **dadurch gekennzeichnet, dass** n = 1 - 4, besonders bevorzugt 1 - 3 ist.

6. Carbodiimide nach einem der Ansprüche 1 bis 5, **dadurch gekennzeichnet, dass** der NCN-Gehalt im Carbodiimid 2 - 10 Gew.-%, bevorzugt 4 - 8 Gew.-%, besonders bevorzugt 5 - 7 Gew.-% beträgt.

7. Carbodiimide nach einem der Ansprüche 1 bis 6, **dadurch gekennzeichnet, dass** diese mittlere Molmassen (Mw) von 1000 - 5000 g/mol, bevorzugt 1500 - 4000 g/mol, besonders bevorzugt 2000 - 3000 g/mol aufweisen.

8. Carbodiimide nach einem der Ansprüche 1 bis 7, **dadurch gekennzeichnet, dass** diese eine Polydispersität D = Mw/Mn von 1,2 - 2, bevorzugt von 1,4 - 1,8, aufweisen.

9. Verfahren zur Herstellung der Carbodiimide nach einem der Ansprüche 1 bis 8 **dadurch**
**gekennzeichnet, dass** Diisocyanate der Formel 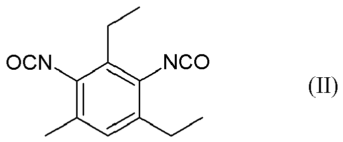 und/oder der Formel 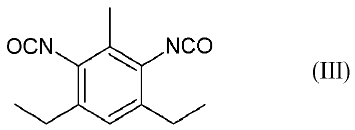 unter Abspaltung von Kohlendioxid bei Temperaturen von 80 °C bis 200 °C in Gegenwart von Katalysatoren und gegebenenfalls Lösungsmittel carbodiimidisiert werden und anschließend die freien NCO-Funktionalitäten mit primären oder sekundären Aminen oder Alkoholen endfunktionalisiert werden.

10. Verfahren nach Anspruch 9 **dadurch gekennzeichnet, dass** die Carbodiimidisierung bei Temperaturen von 80 - 200°C, vorzugsweise 120 - 140 °C, vorzugsweise in Gegenwart eines Katalysators durchgeführt wird, und anschließend gegebenenfalls nach Abdestillation des Katalysators bei Temperaturen von 50 - 120 °C und des nicht umgesetzte Diisocyanats, bei Temperaturen von 160 - 180 °C die restlichen NCO-Gruppen des Carbodiimides mit aliphatischen und/oder aromatischen Aminen, Alkoholen und/oder Alkoxypolyoxyalkylenalkoholen, vorzugsweise Polyethylenmonomethylether und/oder Oleyalkohol, bei Temperaturen von 80 - 140 °C gegebenenfalls in Anwesenheit eines PU-Katalysators umgesetzt werden.

11. Verfahren zur Herstellung der Carbodiimide nach Anspruch 10 **dadurch gekennzeichnet, dass** eine Mischung aus Diisocyanaten der Formel (II) und (III) im Verhältnis 70 : 30 bis 90 : 10 carbodiimidisiert wird.

12. Zusammensetzung enthaltend
- mindestens ein esterbasiertes Polymer
und
- mindestens ein Carbodiimid nach einem der Ansprüche 1 bis 8.

13. Zusammensetzung nach Anspruch 12 **dadurch gekennzeichnet, dass** die Konzentration des Carbodiimides 0,1 - 5 Gew.%, bevorzugt 0,5 - 3 Gew.%, besonders bevorzugt 1 - 2 Gew.% beträgt.

14. Verwendung der Carbodiimide nach einem der Ansprüche 1 bis 8 in esterbasierten Polyolen, in thermoplastischen Polyurethanen (TPU), in Polyurethan-Elastomeren, in PU-Klebstoffen, in PU-Gießharzen, in PU-Schäume oder für PU-Beschichtungen als Schutz gegen hydrolytischen Abbau.
